# EUROPEAN PATENT APPLICATION

(11) **EP 4 349 416 A1**
(43) Date of publication of application: **10.04.2024**
(21) Application number: 22811434.4
(22) Date of filing: 27.05.2022
(51) Int. Cl.: A61Q 13/00, C11D 3/50, A61K 8/37, A61L 9/01, C07C 69/533, C07C 69/54, C07C 69/56, C11B 9/00

(54) **ISOBUTYRIC ACID ESTER COMPOUND HAVING ALKENOYLOXY GROUP AT a-POSITION, PERFUME COMPOSITION, AND USE AS PERFUME**

(30) Priority: 28.05.2021 JP 2021090261
(71) Applicant: MITSUBISHI GAS CHEMICAL COMPANY, INC., Chiyoda-ku Tokyo 100-8324 (JP)
(72) Inventor: YOKOBORI, Umi, Niigata-shi, Niigata 950-3112 (JP); HAMAJIMA, Wataru, Niigata-shi, Niigata 950-3112 (JP); OKAMOTO, Atsushi, Niigata-shi, Niigata 950-3112 (JP)
(74) Representative: Vossius & Partner Patentanwälte Rechtsanwälte mbB
(86) International application number: PCT/JP2022/021844
(87) International publication number: WO 2022/250166

(57) **Abstract**

A fragrance composition contains a compound represented by Formula (1) below as an active ingredient: where, in Formula (1), R¹ is a linear or branched olefinically unsaturated hydrocarbon group having from 2 to 4 carbons, and R² is a linear, branched, or cyclic alkyl group having from 1 to 6 carbons.

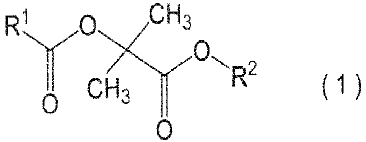

## Description

### Technical Field

The present invention relates to an isobutyrate ester compound having an alkenoyloxy group at the α-position, a fragrance composition, and use as a fragrance.

### Background Art

There are some isobutyrate ester compounds used as fragrances.

For example, Non-Patent Document 1 describes that methyl isobutyrate gives a sweet apricot-like fruity aroma, propyl isobutyrate gives a thick pineapple-like aroma, butyl isobutyrate gives a fresh apple- and banana-like fruity aroma, and isoamyl isobutyrate gives a sweet apricot- and pineapple-like aroma.

Further, Patent Document 1 discloses that an isobutyrate ester compound having a butyryloxy group or a pivaloyloxy group at the α-position has a mint-like aroma or a damascone-like aroma, and can be used as a fragrance and a raw material for a blended fragrance.

Meanwhile, Patent Documents 2 and 3 disclose that isobutyrate esters having an acryloyloxy group or a methacryloyloxy group at the α-position can be used as raw materials for resists. Patent Document 4 discloses that these isobutyrate esters can be used as raw materials for molded materials such as optical lenses. Patent Document 5 discloses that these isobutyrate esters can be used as raw materials for pressure-sensitive adhesives. However, there is no description of the aroma properties of the isobutyrate esters, fragrance compositions containing the isobutyrate esters, and methods of using the isobutyrate esters as fragrances.

### Citation List

### Patent Document

Patent Document 1: WO 2020/004464
Patent Document 2: JP 11-024274 A
Patent Document 3: JP 2017-120403 A
Patent Document 4: WO 2011/051032
Patent Document 5: WO 2014/099300

### Non-Patent Literature

Non-Patent Document 1: "Gosei Koryo: Kagaku to Shohin Chishiki, Zoho Shinban (Synthetic Fragrance: Chemistry and Product Knowledge, New Expanded Edition)", The Chemical Daily Co. Ltd., 2016, p. 580 to 582

### Summary of Invention

### Technical Problem

As described above, there are some isobutyrate ester compounds used as fragrances. In addition, fragrances are used in various fields such as fragrance products, detergents, miscellaneous goods, pharmaceuticals, foods, and the like. In order to increase the value of products, fragrances having a novel aroma are desirable.

Therefore, an object of the present invention is to provide a fragrance composition containing, as an active ingredient, an isobutyrate ester compound useful as a fragrance due to having a complex aroma of a floral aroma and another aroma, wherein the fragrance composition is imparted with strength and diffusibility of aroma in addition to the aroma mentioned above. Another object is to provide an isobutyrate ester compound having a complex aroma of a floral aroma and another aroma, and use of the isobutyrate ester compound as a fragrance.

### Solution to Problem

The present inventors synthesized various compounds and conducted diligent research on the aromas thereof. As a result, the present inventors discovered that the above problems can be solved by a fragrance composition containing, as an active ingredient, a specific isobutyrate ester compound having an alkenoyloxy group at the α-position. The present inventors also found that the above problems can be solved by a specific isobutyrate ester compound having an alkenoyloxy group at the α-position and by using the specific isobutyrate ester compound as a fragrance.

That is, the present invention includes as follows.
<1> A fragrance composition containing a compound represented by Formula (1) below as an active ingredient: 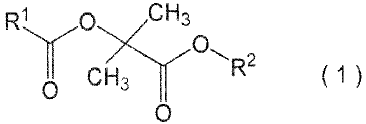 where, in Formula (1), R¹ is a linear or branched olefinically unsaturated hydrocarbon group having from 2 to 4 carbons, and R² is a linear, branched, or cyclic alkyl group having from 1 to 6 carbons.
<2> The fragrance composition according to <1> above, wherein, in Formula (1), R¹ is a linear or branched olefinically unsaturated hydrocarbon group having from 3 to 4 carbons.
<3> The fragrance composition according to <1> or <2> above, wherein, in Formula (1), R¹ is an isopropenyl group.
<4> The fragrance composition according to any one of <1> to <3> above, wherein, in Formula (1), R² is a branched alkyl group having from 3 to 4 carbons.
<5> The fragrance composition according to any one of <1> to <4> above, wherein, in Formula (1), R² is an isopropyl group or a sec-butyl group.
<6> The fragrance composition according to any one of <1> to <5> above, wherein, in Formula (1), R² is an isopropyl group.
<7> The fragrance composition according to <1> above, wherein, in Formula (1), R¹ is an isopropenyl group while R² is an isopropyl group, R¹ is an isopropenyl group while R² is a sec-butyl group, R¹ is a 1-propenyl group while R² is an isopropyl group, R¹ is a 1-propenyl group while R² is a sec-butyl group, R¹ is a 1 -butenyl group while R² is an isopropyl group, R¹ is a 1-methyl-1-propenyl group while R² is an isopropyl group, or R¹ is a 2-methyl-1-propenyl group while R² is an isopropyl group.
<8> Use of a compound represented by Formula (1) below as a fragrance: 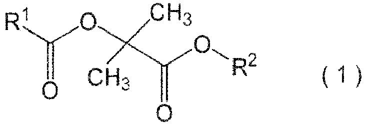 where, in Formula (1), R¹ is a linear or branched olefinically unsaturated hydrocarbon group having from 2 to 4 carbons, and R² is a linear, branched, or cyclic alkyl group having from 1 to 6 carbons.
<9> A compound represented by Formula (2) below: 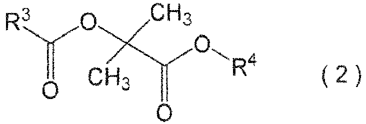 where, in Formula (2), R³ is a linear or branched olefinically unsaturated hydrocarbon group having from 3 to 4 carbons, and R⁴ is a linear, branched, or cyclic alkyl group having from 1 to 6 carbons; however, R³ is not an isopropenyl group.
<10> The compound according to <9> above, wherein, in Formula (2), R⁴ is a branched alkyl group having from 3 to 4 carbons.
<11> The compound according to <9> or <10> above, wherein, in Formula (2), R⁴ is an isopropyl group or a sec-butyl group.
<12> The compound according to any one of <9> to <11> above, wherein, in Formula (2), R⁴ is an isopropyl group.

### Advantageous Effects of Invention

The present invention can provide a fragrance composition containing, as an active ingredient, an isobutyrate ester compound useful as a fragrance due to having a complex aroma of a floral aroma and another aroma, wherein the fragrance composition is imparted with strength and diffusibility of aroma in addition to the aroma mentioned above. An isobutyrate ester compound having a complex aroma of a floral aroma and another aroma, and use of the isobutyrate ester compound as a fragrance can be provided.

### Description of Embodiments

### [Fragrance Composition]

A fragrance composition according to an embodiment of the present invention contains a compound represented by Formula (1) below as an active ingredient.

Hereinafter, the present invention will be described in detail.

### Compound represented by Formula (1)

The compound represented by Formula (1) below is contained as an active ingredient in the fragrance composition according to an embodiment of the present invention. 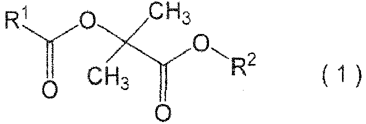 where, in Formula (1), R¹ is a linear or branched olefinically unsaturated hydrocarbon group having from 2 to 4 carbons, and R² is a linear, branched, or cyclic alkyl group having from 1 to 6 carbons.

The compound represented by Formula (1) above has a complex aroma of a floral aroma and another aroma, and can impart, in addition to the aroma described above, strength and diffusibility of aroma to a fragrance composition. In particular, the compound represented by Formula (1) above has a floral aroma and, in addition, exhibits aromas having a green tone, a rosy tone, an herbal tone, a woody tone, a Balsamic tone, a fruity tone and the like at the same time depending on R¹ or R².

In Formula (1), R¹ is a linear or branched olefinically unsaturated hydrocarbon group having from 2 to 4 carbons, preferably a linear or branched olefinically unsaturated hydrocarbon group having from 3 to 4 carbons, and more preferably a linear or branched olefinically unsaturated hydrocarbon group having 3 carbons. R¹ may have an internal carbon-carbon double bond, may have a terminal carbon-carbon double bond, or may have both.

When R¹ has one or more carbon-carbon double bonds, the compound represented by Formula (1) contains any one of the resulting stereoisomers or a mixture of the stereoisomers in any proportion.

Specific examples of R¹ include a vinyl group, a 1-propenyl group, a 2-propenyl group (allyl group), an isopropenyl group, a 1-butenyl group, a 2-butenyl group, a 3-butenyl group, a 1-methyl-1-propenyl group, a l-methyl-2-propenyl group, a 1-methylidene-propyl group, a 2-methyl-1-propenyl group, and a 2-methylidene-propyl group. R¹ is preferably a 1-propenyl group, an isopropenyl group, a 1-butenyl group, a 2-butenyl group, a 1-methyl-1-propenyl group, or a 2-methyl-1-propenyl group, more preferably a 1-propenyl group, an isopropenyl group, a 1-butenyl group, a 1-methyl-1-propenyl group, or a 2-methyl-1-propenyl group, and even more preferably an isopropenyl group.

In the compound represented by Formula (1) above:
R¹ is preferably an isopropenyl group.
R¹ is preferably a 1-propenyl group.
R¹ is preferably a 1-butenyl group.
R¹ is preferably a 1-methyl-1-propenyl group.
R¹ is preferably a 2-methyl-1-propenyl group.

In Formula (1), R² is a linear, branched, or cyclic alkyl group having from 1 to 6 carbons, preferably a linear or branched alkyl group having from 3 to 4 carbons.

When R² has one or more asymmetric carbons, the compound represented by Formula (1) contains any one of the resulting optical isomers or a mixture of the optical isomers in any proportion.

Specific examples of R² include a methyl group, an ethyl group, a n-propyl group, an isopropyl group, a n-butyl group, an isobutyl group (2-methylpropyl group), a sec-butyl group (1-methylpropyl group), a tert-butyl group, a n-pentyl group, a 1-methylbutyl group (2-pentyl group), a 2-methylbutyl group, a 3-methylbutyl group, a neopentyl group (2,2-dimethylpropyl group), a 2-methylbutan-2-yl group, a 1-ethylpropyl group (3-pentyl group), a 3-methylbutan-2-yl group, a n-hexyl group, a 1 -methylpentyl group (2-hexyl group), a 2-methylpentyl group, a 3-methylpentyl group, a 4-methylpentyl group, a 2-methylpentan-2-yl group, a 2,2-dimethylbutyl group, a 3,3-dimethylbutyl group, a 3-methylpentan-2-yl group, a 2,3-dimethylbutyl group, a 4-methylpentan-2-yl group, a 3-hexyl group, a 2-ethylbutyl group, a 2,3-dimethylbutan-2-yl group, a 3,3-dimethylbutan-2-yl group, a 4-methylpentan-3-yl group, a cyclopropyl group, a cyclobutyl group, a cyclopentyl group, and a cyclohexyl group. R² is preferably an isopropyl group or a sec-butyl group, more preferably an isopropyl group.

In the compound represented by Formula (1) above:
R² is preferably an isopropyl group.
R² is preferably a sec-butyl group.

In Formula (1), the combination of R¹ and R² is as follows: preferably, R¹ is a linear or branched olefinically unsaturated hydrocarbon group having from 3 to 4 carbons while R² is a linear or branched alkyl group having from 3 to 4 carbons; more preferably, R¹ is a linear or branched olefinically unsaturated hydrocarbon group having 3 carbons while R² is a linear or branched alkyl group having from 3 to 4 carbons; even more preferably, R¹ is a linear or branched olefinically unsaturated hydrocarbon group having 3 carbons while R² is a branched alkyl group having from 3 to 4 carbons; still more preferably, R¹ is a linear or branched olefinically unsaturated hydrocarbon group having 3 carbons while R² is a branched alkyl group having 3 carbons.

Specifically, the combination of R¹ and R² is more preferably the following: R¹ is one selected from the group consisting of a 1-propenyl group, an isopropenyl group, a 1-butenyl group, a 1-methyl-1-propenyl group, and a 2-methyl-1-propenyl group, while R² is one selected from the group consisting of an isopropyl group and a sec-butyl group.

In the compound represented by Formula (1) above:
Particularly preferably, R¹ is an isopropenyl group while R² is an isopropyl group.
Particularly preferably, R¹ is an isopropenyl group while R² is a sec-butyl group.
Particularly preferably, R¹ is a 1-propenyl group while R² is an isopropyl group.
Particularly preferably, R¹ is a 1-propenyl group while R² is a sec-butyl group.
Particularly preferably, R¹ is a 1-butenyl group while R² is an isopropyl group.
Particularly preferably, R¹ is a 1-methyl-1-propenyl group while R² is an isopropyl group.
Particularly preferably, R¹ is a 2-methyl-1-propenyl group while R² is an isopropyl group.

That is, the fragrance composition according to an embodiment of the present invention particularly preferably contains, as an active ingredient, the compound represented by Formula (1) in which R¹ is an isopropenyl group while R² is an isopropyl group, or R¹ is an isopropenyl group while R² is a sec-butyl group, or R¹ is a 1-propenyl group while R² is an isopropyl group, or R¹ is a 1-propenyl group while R² is a sec-butyl group, or R¹ is a 1-butenyl group while R² is an isopropyl group, or R¹ is a 1-methyl-1-propenyl group while R² is an isopropyl group, or R¹ is a 2-methyl-1-propenyl group while R² is an isopropyl group.

In an embodiment of the present invention, the compound represented by Formula (1) is exemplified by a compound represented by any of Formulas (1-1) to (I-96) below. Among these, the compound represented by Formula (1) is preferably a compound represented by any of Formulas (1-12), (1-16), (1-28), (1-32), (1-36), (1-60), and (1-84) below.

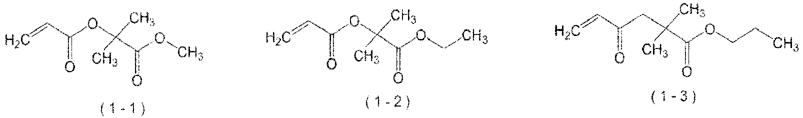

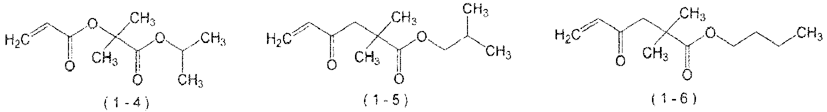

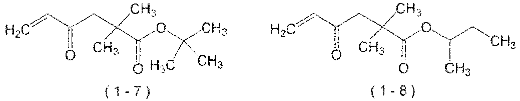

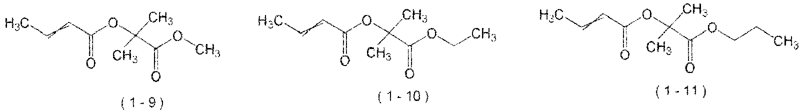

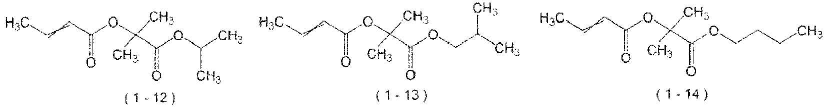

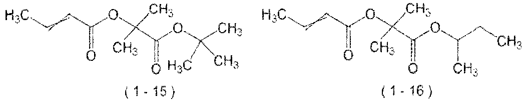

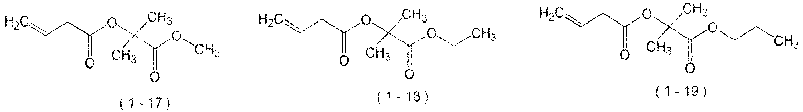

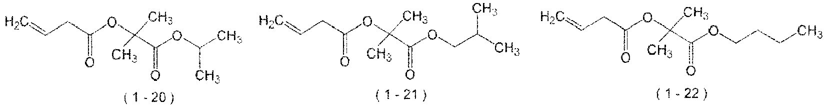

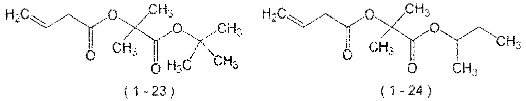

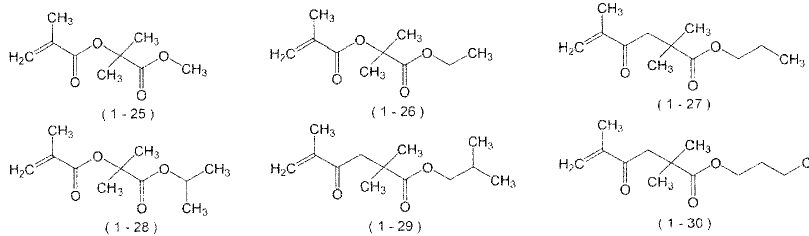

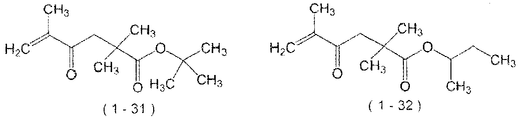

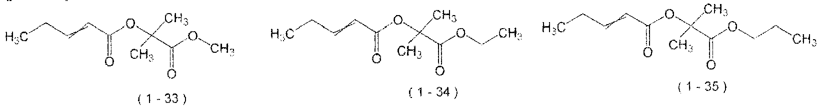

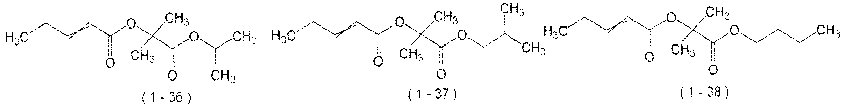

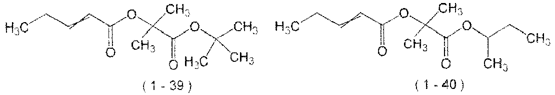

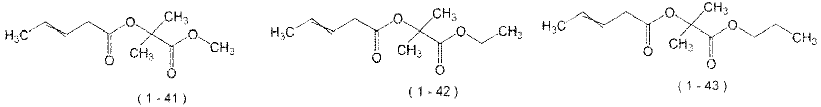

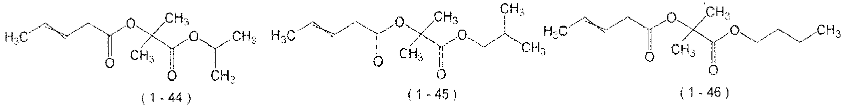

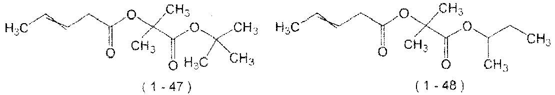

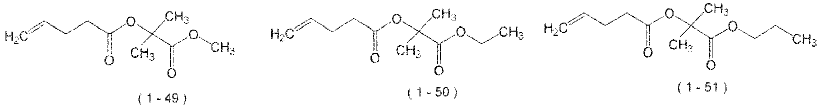

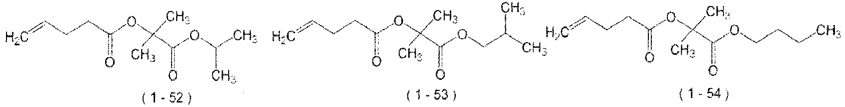

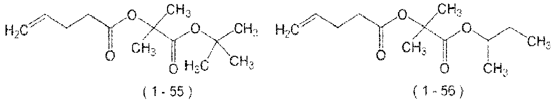

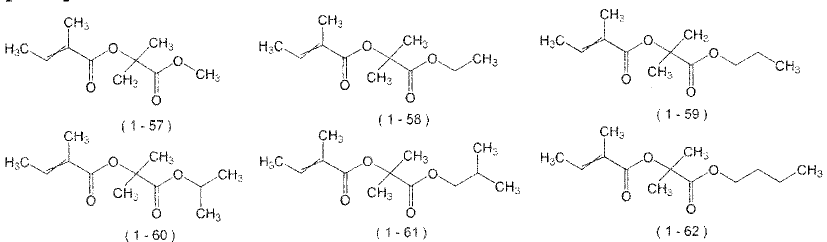

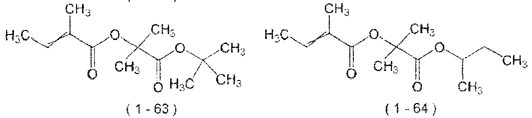

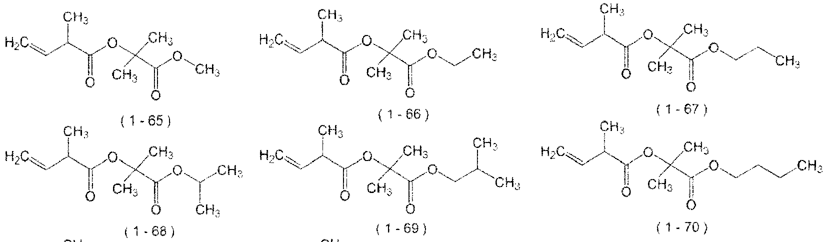

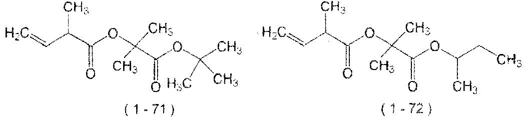

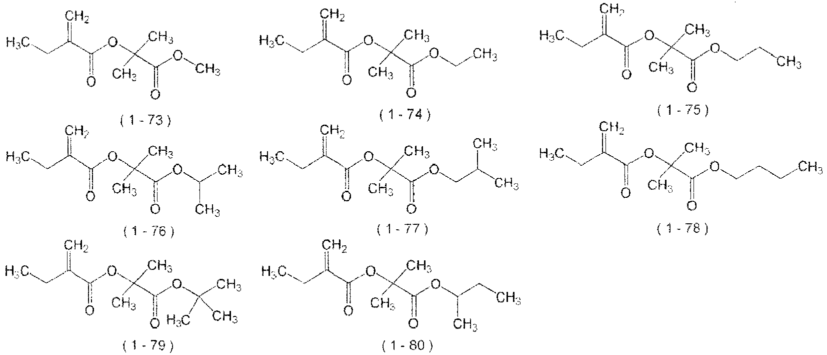

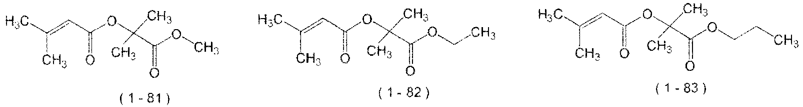

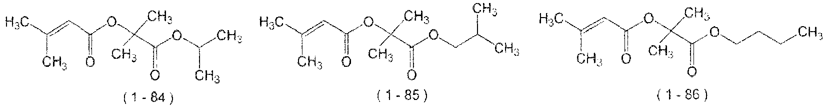

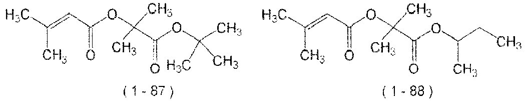

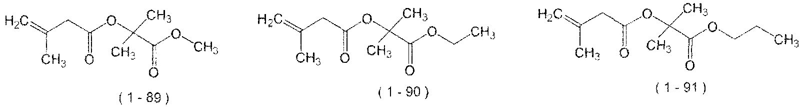

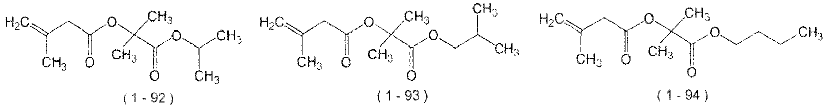

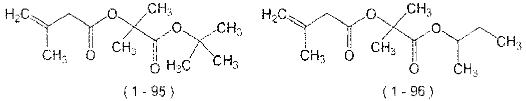

The carbon-carbon double bond represented by the crossed double line in Formula (1-9), Formula (1-10), Formula (1-11), Formula (1-12), Formula (1-13), Formula (1-14), Formula (1-15), Formula (1-16), Formula (1-33), Formula (1-34), Formula (1-35), Formula (1-36), Formula (1-37), Formula (1-38), Formula (1-39), Formula (1-40), Formula (1-41), Formula (1-42), Formula (1-43), Formula (1-44), Formula (1-45), Formula (1-46), Formula (1-47), Formula (1-48), Formula (1-57), Formula (1-58), Formula (1-59), Formula (1-60), Formula (1-61), Formula (1-62), Formula (1-63) and Formula (1-64) represents both the trans (E) and cis (Z) stereoisomers resulting from the double bond.

The compound represented by Formula (1) has a complex aroma of a floral aroma and another aroma. As such, adding the compound represented by Formula (1) as an active ingredient of the fragrance composition can impart a complex aroma of a floral aroma and another aroma. Furthermore, the compound represented by Formula (1) can impart strength and diffusibility of aroma to a fragrance composition.

In particular, the compound represented by Formula (1) above has a floral aroma and, in addition, exhibits aromas having a green tone, a rosy tone, an herbal tone, a woody tone, a Balsamic tone, a fruity tone and the like at the same time depending on R¹ or R². As such, adding the compound represented by Formula (1) as an active ingredient of a fragrance composition can impart aromas having a green tone, a rosy tone, an herbal tone, a woody tone, a Balsamic tone, a fruity tone and the like at the same time in addition to the floral aroma. Furthermore, the compound represented by Formula (1) can impart strength and diffusibility of aroma to a fragrance composition.

The fragrance composition containing the compound represented by Formula (1) above as an active ingredient is a fragrance composition imparted with strength and diffusibility of aroma in addition to the aroma described above.

The "active ingredient" in the fragrance composition refers to a component that changes the aroma of the fragrance composition, particularly refers to a component that changes the aroma of the fragrance composition to a preferable fragrance note, and refers to a component having a property of improving the aroma of the fragrance composition.

The content of the compound represented by Formula (1) in the fragrance composition may be changed as appropriate depending on the type of compound as well as the type and strength of the desired aroma. The content of the compound represented by Formula (1) in the fragrance composition is preferably 0.001 mass% or greater, more preferably 0.01 mass% or greater, and even more preferably 0.1 mass% or greater. Also, the content of the compound represented by Formula (1) in the fragrance composition is preferably 90 mass% or less, more preferably 70 mass% or less, and even more preferably 50 mass% or less.

### <Composition of Fragrance Composition and Additional Component>

The fragrance composition according to an embodiment of the present invention contains the compound represented by Formula (1) as an active ingredient. The fragrance composition is any composition containing at least one compound represented by Formula (1) and is not particularly limited and may contain two or more compounds represented by Formula (1).

An additional component of the fragrance composition according to an embodiment of the present invention is not limited as long as the fragrance composition contains the compound represented by Formula (1) as an active ingredient. However, the fragrance composition according to an embodiment of the present invention preferably further contains a fragrance in addition to the compound represented by Formula (1).

Note that the term "fragrance composition" refers to a composition to be added to various perfumes and cosmetics, pharmaceutical products, foods, beverages, and the like to impart an aroma to these products, or a composition used per se as a perfume or the like. The fragrance composition may contain an additive, such as a solvent, as necessary in addition to the compound represented by Formula (1) and a fragrance other than the compound represented by Formula (1).

Examples of the additional component contained in the fragrance composition according to an embodiment of the present invention in addition to the compound represented by Formula (1) include fragrances other than the compound represented by Formula (1), surfactants, solvents, antioxidants, and coloring agents. The additional component is preferably at least one selected from the group consisting of fragrances other than the compound represented by Formula (1), surfactants, solvents, antioxidants, and coloring agents, and more preferably at least one selected from the group consisting of fragrances other than the compound represented by Formula (1) and solvents.

The inclusion of a fragrance other than the compound represented by Formula (1) allows the aroma to be adjusted to accommodate a target product. In addition, the inclusion of a solvent allows for easy dissolution in and impregnation of a target product, making it possible to adjust the intensity of the aroma or the durability of the aroma.

The fragrance other than the compound represented by Formula (1) is not limited as long as it is a known fragrance component, and a wide range of fragrances can be used. For example, one, or two or more of the following fragrances can be selected and used at any mixing ratio.

Examples of the fragrance other than the compound represented by Formula (1) include hydrocarbons, alcohols, phenols, esters, aldehydes, ketones, acetals, ketals, ethers, nitriles, lactones, natural essential oils, and natural extracts.

Examples of hydrocarbons include limonene, α-pinene, β-pinene, terpinene, cedrene, longifolene, and valencene.

Examples of alcohols include linalool, citronellol, geraniol, nerol, terpineol, dihydromyrcenol, ethyllinalool, farnesol, nerolidol, cis-3-hexenol, cedrol, menthol, borneol, β-phenylethyl alcohol, benzyl alcohol, phenyl hexanol, 2,2,6-trimethylcyclohexyl-3-hexanol, 1-(2-t-butylcyclohexyloxy)-2-butanol, 4-isopropylcyclohexane methanol, 4-t-butylcyclohexanol, 4-methyl-2-(2-methylpropyl)tetrahydro-2H-pyran-4-ol, 2-methyl-4-(2,2,3-trimethyl-3-cyclopenten-1-yl)-2-buten-1-ol, 2-ethyl-4-(2,2,3-trimethyl-3-cyclopenten-1-yl)-2-buten-1-ol, isocamphylcyclohexanol, and 3,7-dimethyl-7-methoxyoctan-2-ol.

Examples of phenols include eugenol, thymol, and vanillin.

Examples of esters include linalyl formate, citronellyl formate, geranyl formate, n-hexyl acetate, cis-3-hexenyl acetate, linalyl acetate, citronellyl acetate, geranyl acetate, nellyl acetate, terpinyl acetate, nopil acetate, bornyl acetate, isobomyl acetate, o-t-butylcyclohexyl acetate, p-t-butylcyclohexyl acetate, tricyclodecenyl acetate, benzyl acetate, styralyl acetate, cinnamyl acetate, dimethylbenzyl carbinyl acetate, 3-pentyltetrahydropyran-4-yl acetate, citronellyl propionate, tricyclodecenyl propionate, allylcyclohexyl propionate, ethyl-2-cyclohexyl propionate, benzyl propionate, citronellyl butyrate, dimethylbenzylcarbinyl n-butyrate, tricyclodecenyl isobutyrate, methyl-2-nonenoate, methyl benzoate, benzyl benzoate, methyl cinnamate, methyl salicylate, n-hexyl salicylate, cis-3-hexenyl salicylate, geranyl tiglate, cis-3-hexenyl tiglate, methyl jasmonate, methyldihydro jasmonate, methyl-2,4-dihydroxy-3,6-dimethyl benzoate, ethylmethylphenyl glycidate, methyl anthranilate, and fruitate.

Examples of aldehydes include n-octanal, n-decanal, n-dodecanal, 2-methylundecanal, 10-undecenal, citronellal, citral, hydroxycitronellal, dimethyl tetrahydrobenzaldehyde, 4(3)-(4-hydroxy-4-methylpentyl)-3-cyclohexene-1-carboaldehyde, 2-cyclohexyl propanal, p-t-butyl-α-methylhydrocinnamic aldehyde, p-isopropyl-α-methylhydrocinnamic aldehyde, p-ethyl-α,α-dimethylhydrocinnamic aldehyde, α-amylcinnamic aldehyde, α-hexylcinnamic aldehyde, piperonal, and α-methyl-3,4-methylenedioxyhydrocinnamic aldehyde.

Examples of ketones include methylheptenone, 4-methylene-3,5,6,6-tetramethyl-2-heptanone, amylcyclopentanone, 3-methyl-2-(cis-2-penten-1-yl)-2-cyclopenten-1-one, methylcyclopentenolone, rose ketone, γ-methylionone, α-ionone, carbone, menthone, camphor, nootkatone, benzylacetone, anysilacetone, methyl-β-naphthylketone, 2,5-dimethyl-4-hydroxy-3(2H)-furanone, maltol, 7-acetyl-1,2,3,4,5,6,7,8-octahydro-1,1,6,7-tetramethyl naphthalene, muscone, civetone, cyclopentadecanone, and cyclohexedecenone.

Examples of acetals and ketals include acetoaldehyde ethylphenylpropyl acetal, citraldiethyl acetal, phenylacetoaldehyde glycerin acetal, and ethylacetoacetate ethylene glycol ketal.

Examples of ethers include anetol, β-naphthylmethyl ether, β-naphthylethyl ether, limonene oxide, rose oxide, 1,8-cineol, racemic or optically active dodecahydro-3a,6,6,9a-tetramethylnaphtho[2,1-b]furane.

Examples of nitriles include citronellyl nitrile.

Examples of lactones include γ-nonalactone, γ-undecalactone, σ-decalactone, γ-jasmolactone, cumarin, cyclopentadecanolide, cyclohexadecanolide, ambrettolide, ethylene brassylate, and 11 -oxahexadecanolide.

Examples of natural essential oils and natural extracts include those of orange, lemon, bergamot, mandarin, peppermint, spearmint, lavender, chamomile, rosemary, eucalyptus, sage, basil, rose, geranium, jasmine, ylang-ylang, anise, clove, ginger, nutmeg, cardamom, cedar, Japanese cypress, sandalwood, vetiver, patchouli, and labdanum.

Examples of the solvent include dipropylene glycol, diethyl phthalate, ethylene glycol, propylene glycol, methyl myristate, and triethyl citrate.

Examples of the surfactant include polyoxyethylene lauryl sulfate ether.

The fragrance composition containing the compound represented by Formula (1) as an active ingredient can be used as an aroma ingredient for various products.

The products in which the fragrance composition can be used include: fragrance products, such as perfumes and colognes; cosmetics for hair, such as shampoos, conditioners, hair tonics, hair creams, mousses, gels, pomades, and sprays; cosmetics for skin, such as face lotions, serums, creams, emulsions, facial masks, foundations, face powders, lipsticks, and various types of makeup; various detergents for health and sanitation, such as dish detergents, laundry detergents, softeners, disinfectant detergents, odor eliminating detergents, indoor fragrances, furniture care products, glass cleaners, furniture cleaners, floor cleaners, disinfectants, insecticides, bleaches, germicides, and repellents; quasi-drugs, such as toothpaste, mouthwash, bath salts, antiperspirants, and perm solutions; miscellaneous goods such as toilet paper and tissue paper; pharmaceuticals; and food products.

The amount of fragrance composition added to the products described above is not limited and can be selected depending on the type, nature, and sensory effect of the product to be perfumed. For example, the amount of fragrance composition added to the products is preferably 0.00001 mass% or greater, more preferably 0.0001 mass% or greater, and even more preferably 0.001 mass% or greater. Also, the amount of the fragrance composition added to the products is preferably 80 mass% or less, more preferably 60 mass% or less, and even more preferably 40 mass% or less.

Note that, when the fragrance composition is used as an aromatic oil, perfume, or the like, the amount of fragrance composition added to the products may be 80 mass% or greater, or may be 100 mass%.

### Use of Compound Represented by Formula (1) as Fragrance

The compound represented by Formula (1) below can be used as a fragrance. An embodiment of the present invention also provides the use of the compound represented by Formula (1) as a fragrance. 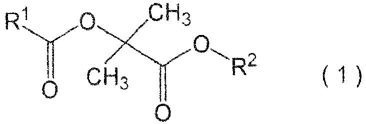 where, in Formula (1), R¹ is a linear or branched olefinically unsaturated hydrocarbon group having from 2 to 4 carbons, and R² is a linear, branched, or cyclic alkyl group having from 1 to 6 carbons.

The compound represented by Formula (1) above has a complex aroma of a floral aroma and another aroma. Thus, the compound represented by Formula (1) above can be used as a fragrance. In particular, the compound represented by Formula (1) above has a floral aroma and, in addition, exhibits aromas having a green tone, a rosy tone, an herbal tone, a woody tone, a Balsamic tone, a fruity tone and the like at the same time depending on R¹ or R². Thus, the compound represented by Formula (1) above can be used as a fragrance.

The compound represented by Formula (1) that can be used as a fragrance is the same as the compound described in the section of "Compound Represented by Formula (1)" in "Fragrance Composition", and the preferred structures are also the same. The details are as follows.

In Formula (1), R¹ is a linear or branched olefinically unsaturated hydrocarbon group having from 2 to 4 carbons, preferably a linear or branched olefinically unsaturated hydrocarbon group having from 3 to 4 carbons, and more preferably a linear or branched olefinically unsaturated hydrocarbon group having 3 carbons. R¹ may have an internal carbon-carbon double bond, may have a terminal carbon-carbon double bond, or may have both.

When R¹ has one or more carbon-carbon double bonds, the compound represented by Formula (1) contains any one of the resulting stereoisomers or a mixture of the stereoisomers in any proportion.

Specific examples of R¹ include a vinyl group, a 1-propenyl group, a 2-propenyl group (allyl group), an isopropenyl group, a 1-butenyl group, a 2-butenyl group, a 3-butenyl group, a 1-methyl-1-propenyl group, a 1-methyl-2-propenyl group, a 1-methylidene-propyl group, a 2-methyl-1-propenyl group, and a 2-methylidene-propyl group. R¹ is preferably a 1-propenyl group, an isopropenyl group, a 1-butenyl group, a 2-butenyl group, a 1-methyl-1-propenyl group, or a 2-methyl-1-propenyl group, more preferably a 1-propenyl group, an isopropenyl group, a 1-butenyl group, a 1-methyl-1-propenyl group, or a 2-methyl-1-propenyl group, and even more preferably an isopropenyl group.

In the compound represented by Formula (1) above:
R¹ is preferably an isopropenyl group.
R¹ is preferably a 1-propenyl group.
R¹ is preferably a 1-butenyl group.
R¹ is preferably a 1-methyl-1-propenyl group.
R¹ is preferably a 2-methyl-1-propenyl group.

In Formula (1), R² is a linear, branched, or cyclic alkyl group having from 1 to 6 carbons, preferably a linear or branched alkyl group having from 3 to 4 carbons.

When R² has one or more asymmetric carbons, the compound represented by Formula (1) contains any one of the resulting optical isomers or a mixture of the optical isomers in any proportion.

Specific examples of R² include a methyl group, an ethyl group, a n-propyl group, an isopropyl group, a n-butyl group, an isobutyl group (2-methylpropyl group), a sec-butyl group (1-methylpropyl group), a tert-butyl group, a n-pentyl group, a 1-methylbutyl group (2-pentyl group), a 2-methylbutyl group, a 3-methylbutyl group, a neopentyl group (2,2-dimethylpropyl group), a 2-methylbutan-2-yl group, a 1-ethylpropyl group (3-pentyl group), a 3-methylbutan-2-yl group, a n-hexyl group, a 1-methylpentyl group (2-hexyl group), a 2-methylpentyl group, a 3-methylpentyl group, a 4-methylpentyl group, a 2-methylpentan-2-yl group, a 2,2-dimethylbutyl group, a 3,3-dimethylbutyl group, a 3-methylpentan-2-yl group, a 2,3-dimethylbutyl group, a 4-methylpentan-2-yl group, a 3-hexyl group, a 2-ethylbutyl group, a 2,3-dimethylbutan-2-yl group, a 3,3-dimethylbutan-2-yl group, a 4-methylpentan-3-yl group, a cyclopropyl group, a cyclobutyl group, a cyclopentyl group, and a cyclohexyl group. R² is preferably an isopropyl group or a sec-butyl group, more preferably an isopropyl group.

In the compound represented by Formula (1) above:
R² is preferably an isopropyl group.
R² is preferably a sec-butyl group.

In Formula (1), the combination of R¹ and R² is as follows: preferably, R¹ is a linear or branched olefinically unsaturated hydrocarbon group having from 3 to 4 carbons while R² is a linear or branched alkyl group having from 3 to 4 carbons; more preferably, R¹ is a linear or branched olefinically unsaturated hydrocarbon group having 3 carbons while R² is a linear or branched alkyl group having from 3 to 4 carbons; even more preferably, R¹ is a linear or branched olefinically unsaturated hydrocarbon group having 3 carbons while R² is a branched alkyl group having from 3 to 4 carbons; still more preferably, R¹ is a linear or branched olefinically unsaturated hydrocarbon group having 3 carbons while R² is a branched alkyl group having 3 carbons.

Specifically, the combination of R¹ and R² is more preferably the following: R¹ is one selected from the group consisting of a 1-propenyl group, an isopropenyl group, a 1-butenyl group, a 1-methyl-1-propenyl group, and a 2-methyl-1-propenyl group, while R² is one selected from the group consisting of an isopropyl group and a sec-butyl group.

In the compound represented by Formula (1) above:
Particularly preferably, R¹ is an isopropenyl group while R² is an isopropyl group.
Particularly preferably, R¹ is an isopropenyl group while R² is a sec-butyl group.
Particularly preferably, R¹ is a 1-propenyl group while R² is an isopropyl group.
Particularly preferably, R¹ is a 1-propenyl group while R² is a sec-butyl group.
Particularly preferably, R¹ is a 1-butenyl group while R² is an isopropyl group.
Particularly preferably, R¹ is a 1-methyl-1-propenyl group while R² is an isopropyl group.
Particularly preferably, R¹ is a 2-methyl-1-propenyl group while R² is an isopropyl group.

That is, the compound represented by Formula (1) in which R¹ is an isopropenyl group while R² is an isopropyl group, or R¹ is an isopropenyl group while R² is a sec-butyl group, or R¹ is a 1-propenyl group while R² is an isopropyl group, or R¹ is a 1-propenyl group while R² is a sec-butyl group, or R¹ is a 1-butenyl group while R² is an isopropyl group, or R¹ is a 1-methyl-1-propenyl group while R² is an isopropyl group, or R¹ is a 2-methyl-1-propenyl group while R² is an isopropyl group is particularly preferably used as a fragrance.

### [Compound Represented by Formula (2)]

A compound according to an embodiment of the present invention is represented by Formula (2) below. 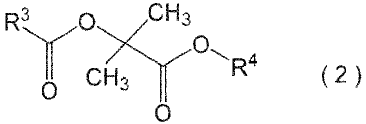 where, in Formula (2), R³ is a linear or branched olefinically unsaturated hydrocarbon group having from 3 to 4 carbons, and R⁴ is a linear, branched, or cyclic alkyl group having from 1 to 6 carbons; however, R³ is not an isopropenyl group.

The compound represented by Formula (2) above has a complex aroma of a floral aroma and another aroma. Thus, the compound represented by Formula (2) above is useful as a fragrance. In particular, the compound represented by Formula (2) above has a floral aroma and, in addition, exhibits aromas having a green tone, a rosy tone, an herbal tone, a woody tone, a Balsamic tone, a fruity tone and the like at the same time depending on R³ or R⁴. Thus, the compound represented by Formula (2) above is useful as a fragrance.

In Formula (2), R³ is a linear or branched olefinically unsaturated hydrocarbon group having from 3 to 4 carbons that is not an isopropenyl group, and more preferably a linear or branched olefinically unsaturated hydrocarbon group having 3 carbons. R' may have an internal carbon-carbon double bond, may have a terminal carbon-carbon double bond, or may have both.

When R³ has one or more carbon-carbon double bonds, the compound represented by Formula (2) contains any one of the resulting stereoisomers or a mixture of the stereoisomers in any proportion.

Specific examples of R³ include a 1-propenyl group, a 2-propenyl group (allyl group), a 1-butenyl group, a 2-butenyl group, a 3-butenyl group, a 1-methyl-1-propenyl group, a 1-methyl-2-propenyl group, a 1-methylidene-propyl group, a 2-methyl-1-propenyl group, and a 2-methylidene-propyl group; R³ is preferably a 1-propenyl group, a 1-butenyl group, a 1-methyl-1-propenyl group, or a 2-methyl-1-propenyl group.

In the compound represented by Formula (2) above:
Preferably, R³ is a 1-propenyl group.
Preferably, R³ is a 1-butenyl group.
Preferably, R³ is a 1-methyl-1-propenyl group.
Preferably, R³ is a 2-methyl-1-propenyl group.

In Formula (2), R⁴ is a linear, branched, or cyclic alkyl group having from 1 to 6 carbons, preferably a linear or branched alkyl group having from 3 to 4 carbons.

When R⁴ has one or more asymmetric carbons, the compound represented by Formula (2) contains any one of the resulting optical isomers or a mixture of the optical isomers in any proportion.

Specific examples of R⁴ include a methyl group, an ethyl group, a n-propyl group, an isopropyl group, a n-butyl group, an isobutyl group (2-methylpropyl group), a sec-butyl group (1-methylpropyl group), a tert-butyl group, a n-pentyl group, a 1-methylbutyl group (2-pentyl group), a 2-methylbutyl group, a 3-methylbutyl group, a neopentyl group (2,2-dimethylpropyl group), a 2-methylbutan-2-yl group, a 1-ethylpropyl group (3-pentyl group), a 3-methylbutan-2-yl group, a n-hexyl group, a 1 -methylpentyl group (2-hexyl group), a 2-methylpentyl group, a 3-methylpentyl group, a 4-methylpentyl group, a 2-methylpentan-2-yl group, a 2,2-dimethylbutyl group, a 3,3-dimethylbutyl group, a 3-methylpentan-2-yl group, a 2,3-dimethylbutyl group, a 4-methylpentan-2-yl group, a 3-hexyl group, a 2-ethylbutyl group, a 2,3-dimethylbutan-2-yl group, a 3,3-dimethylbutan-2-yl group, a 4-methylpentan-3-yl group, a cyclopropyl group, a cyclobutyl group, a cyclopentyl group, and a cyclohexyl group; R⁴ is preferably an isopropyl group, a sec-butyl group, or an isobutyl group, more preferably an isopropyl group or a sec-butyl group, and even more preferably an isopropyl group.

In the compound represented by Formula (2) above:
Preferably, R⁴ is an isopropyl group.
Preferably, R⁴ is a sec-butyl group.

In Formula (2), the combination of R³ and R⁴ is as follows: preferably, R³ is a linear or branched olefinically unsaturated hydrocarbon group having from 3 to 4 carbons while R⁴ is a linear or branched alkyl group having from 3 to 4 carbons; more preferably, R³ is a linear or branched olefinically unsaturated hydrocarbon group having 3 carbons while R⁴ is a linear or branched alkyl group having from 3 to 4 carbons; even more preferably, R³ is a linear or branched olefinically unsaturated hydrocarbon group having 3 carbons while R⁴ is a branched alkyl group having from 3 to 4 carbons; still more preferably, R³ is a linear or branched olefinically unsaturated hydrocarbon group having 3 carbons while R⁴ is a branched alkyl group having 3 carbons.

Specifically, the combination of R³ and R⁴ is more preferably the following: R³ is one selected from the group consisting of a 1-propenyl group, an isopropenyl group, a 1-butenyl group, a 1-methyl-1-propenyl group, and a 2-methyl-1-propenyl group, while R⁴ is one selected from the group consisting of an isopropyl group and a sec-butyl group.

In the compound represented by Formula (2) above:
Particularly preferably, R³ is a 1-propenyl group while R⁴ is an isopropyl group.
Particularly preferably, R³ is a 1-propenyl group while R⁴ is a sec-butyl group.
Particularly preferably, R³ is a 1-butenyl group while R⁴ is an isopropyl group.
Particularly preferably, R³ is a 1-methyl-1-propenyl group while R⁴ is an isopropyl group.
Particularly preferably, R³ is a 2-methyl-1-propenyl group while R⁴ is an isopropyl group.

In an embodiment of the present invention, the compound represented by Formula (2) is exemplified by a compound represented by any of Formulas (2-1) to (2-80) below. Among these, the compound represented by Formula (2) is preferably a compound represented by any of Formulas (2-4), (2-8), (2-20), (2-44), and (2-68) below.

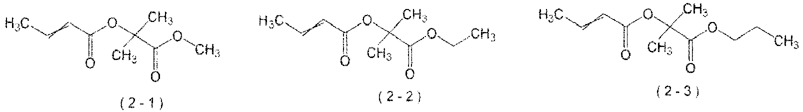

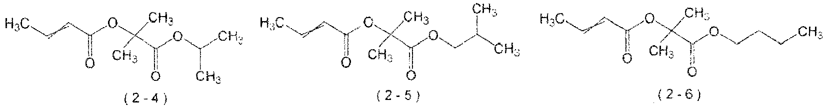

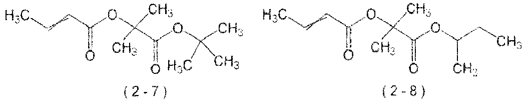

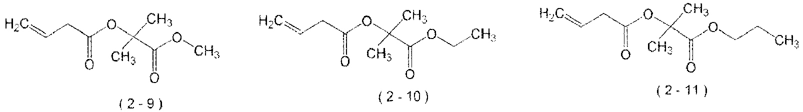

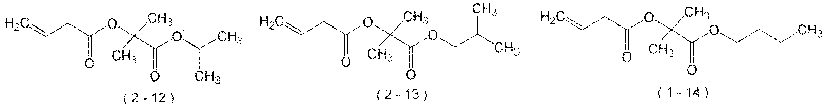

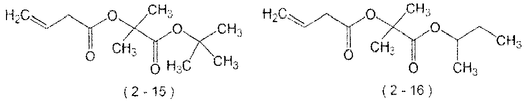

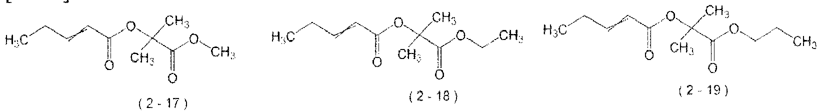

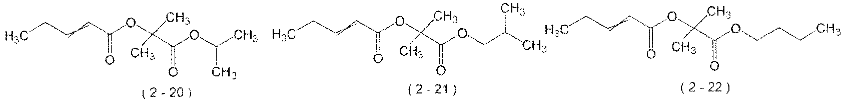

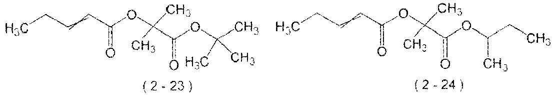

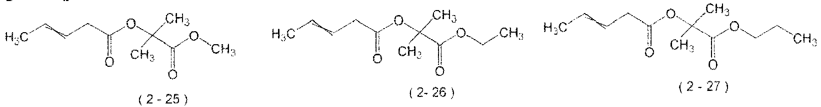

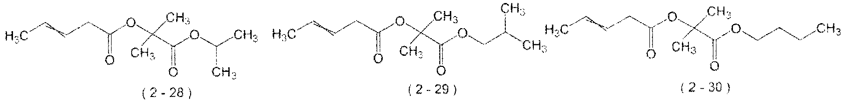

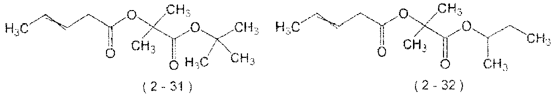

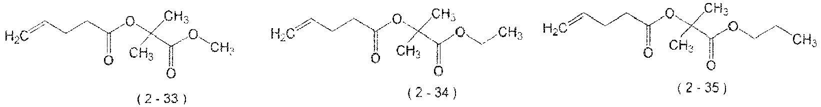

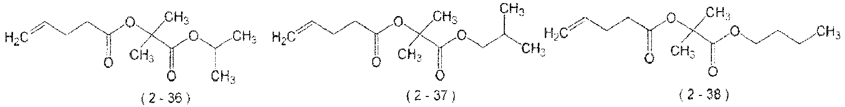

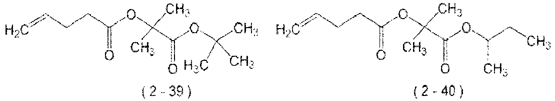

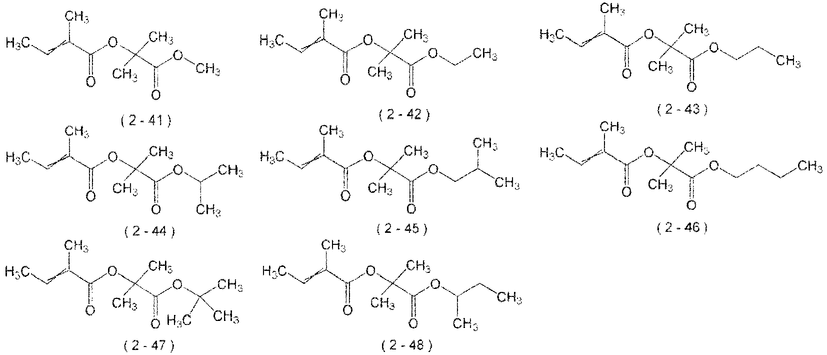

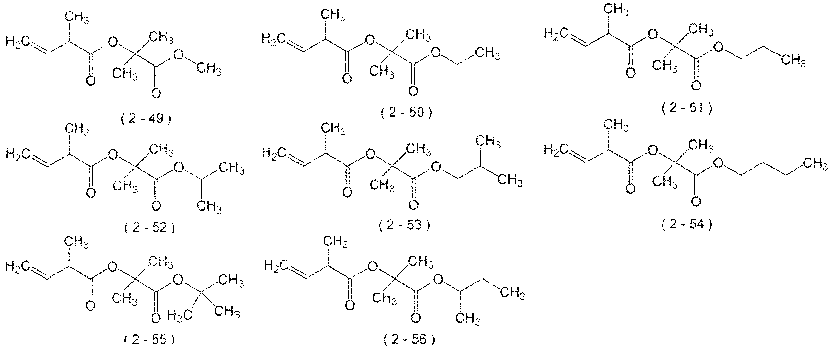

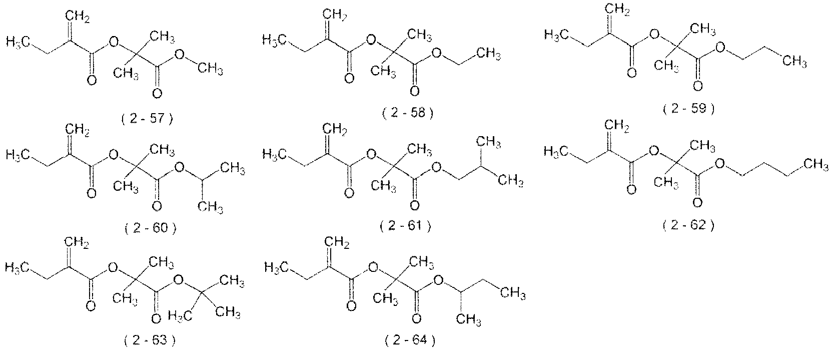

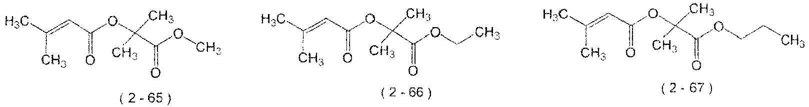

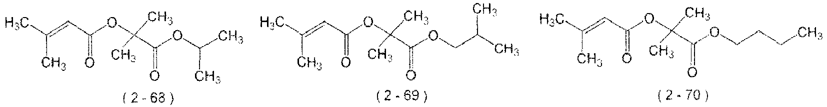

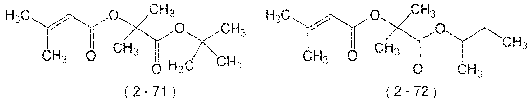

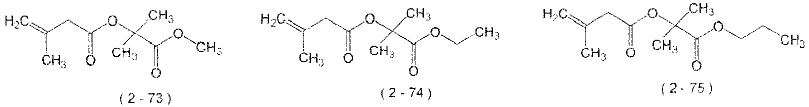

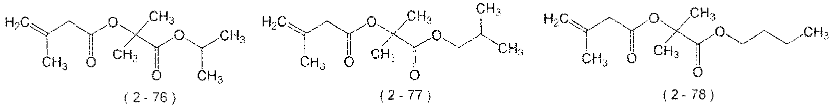

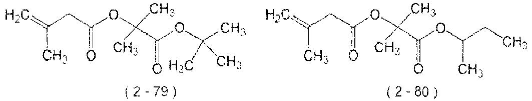

The carbon-carbon double bond represented by the crossed double line in Formula (2-1), Formula (2-2), Formula (2-3), Formula (2-4), Formula (2-6), Formula (2-7), Formula (2-8), Formula (2-17), Formula (2-18), Formula (2-19), Formula (2-20), Formula (2-21), Formula (2-22), Formula (2-23), Formula (2-24), Formula (2-25), Formula (2-26), Formula (2-27), Formula (2-28), Formula (2-29), Formula (2-30), Formula (2-31), Formula (2-32), Formula (2-41), Formula (2-42), Formula (2-43), Formula (2-44), Formula (2-45), Formula (2-46), Formula (2-47), and Formula (2-48) represents both the trans (E) and cis (Z) stereoisomers resulting from the double bond.

The compound represented by Formula (2) has a complex aroma of a floral aroma and another aroma. As such, the compound represented by Formula (2) above is useful as a fragrance.

In particular, the compound represented by Formula (2) has a floral aroma and, in addition, exhibits aromas having a green tone, a rosy tone, an herbal tone, a woody tone, a Balsamic tone, a fruity tone and the like at the same time depending on R³ or R⁴. Thus, the compound represented by Formula (2) is useful as a fragrance.

The compound represented by Formula (2) can be used as an aroma ingredient for various products.

The products in which the compound represented by Formula (2) can be used as an aroma ingredient include: fragrance products, such as perfumes and colognes; cosmetics for hair, such as shampoos, conditioners, hair tonics, hair creams, mousses, gels, pomades, and sprays; cosmetics for skin, such as face lotions, serums, creams, emulsions, facial masks, foundations, face powders, lipsticks, and various types of makeup; various detergents for health and sanitation, such as dish detergents, laundry detergents, softeners, disinfectant detergents, odor eliminating detergents, indoor fragrances, furniture care products, glass cleaners, furniture cleaners, floor cleaners, disinfectants, insecticides, bleaches, germicides, and repellents; quasi-drugs, such as toothpaste, mouthwash, bath salts, antiperspirants, and perm solutions; miscellaneous goods such as toilet paper and tissue paper; pharmaceuticals; and food products.

The amount of the compound represented by Formula (2) added to the products described above is not limited and can be selected depending on the type, nature, and sensory effect of the product to be perfumed. For example, the amount of the compound represented by Formula (2) added to the products is preferably 0.00001 mass% or greater, more preferably 0.0001 mass% or greater, and even more preferably 0.001 mass% or greater. Also, the amount of the compound represented by Formula (2) added to the products is preferably 80 mass% or less, more preferably 60 mass% or less, and even more preferably 40 mass% or less.

Note that, when the compound represented by Formula (2) is used as an aromatic oil, perfume, or the like, the amount of the compound represented by Formula (2) added to the products may be 80 mass% or greater, or may be 100 mass%.

### <Fragrance Composition Containing Compound Represented by Formula (2) as Active Ingredient>

The compound represented by Formula (2) below according to an embodiment of the present invention is also useful as an active ingredient of a fragrance composition. Hereinafter, a fragrance composition containing the compound represented by Formula (2) as an active ingredient will be described. 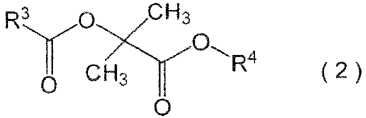 where, in Formula (2), R³ is a linear or branched olefinically unsaturated hydrocarbon group having from 3 to 4 carbons, and R⁴ is a linear, branched, or cyclic alkyl group having from 1 to 6 carbons; however, R³ is not an isopropenyl group.

The compound represented by Formula (2) has a complex aroma of a floral aroma and another aroma. As such, adding the compound represented by Formula (2) as an active ingredient of a fragrance composition can impart a complex aroma of a floral aroma and another aroma. Furthermore, the compound represented by Formula (2) can impart strength and diffusibility of aroma to a fragrance composition.

In particular, the compound represented by Formula (2) above has a floral aroma and, in addition, exhibits aromas having a green tone, a rosy tone, an herbal tone, a woody tone, a Balsamic tone, a fruity tone and the like at the same time depending on R³ or R⁴. As such, adding the compound represented by Formula (2) as an active ingredient of a fragrance composition can impart aromas having a green tone, a rosy tone, an herbal tone, a woody tone, a Balsamic tone, a fruity tone and the like at the same time in addition to the floral aroma. Furthermore, the compound represented by Formula (2) can impart strength and diffusibility of aroma to a fragrance composition.

The fragrance composition containing the compound represented by Formula (2) above as an active ingredient is a fragrance composition imparted with strength and diffusibility of aroma in addition to the aroma described above.

The "active ingredient" in the fragrance composition refers to a component that changes the aroma of the fragrance composition, particularly refers to a component that changes the aroma of the fragrance composition to a preferable fragrance note, and refers to a component having a property of improving the aroma of the fragrance composition.

The compound represented by Formula (2) that can be used as an active ingredient in the fragrance composition is the same as the compound represented by Formula (2) described above, and the preferred structures are also the same.

The content of the compound represented by Formula (2) in the fragrance composition may be changed as appropriate depending on the type of compound as well as the type and strength of the desired aroma. The content of the compound represented by Formula (2) in the fragrance composition is preferably 0.001 mass% or greater, more preferably 0.01 mass% or greater, and even more preferably 0.1 mass% or greater. Also, the content of the compound represented by Formula (2) in the fragrance composition is preferably 90 mass% or less, more preferably 70 mass% or less, and even more preferably 50 mass% or less.

### (Composition of Fragrance Composition and Additional Component)

The fragrance composition containing the compound represented by Formula (2) as an active ingredient is not limited as long as it contains at least one compound represented by Formula (2), and may contain two or more compounds represented by Formula (2).

An additional component of the fragrance composition containing the compound represented by Formula (2) as an active ingredient is not limited as long as the fragrance composition contains the compound represented by Formula (2) as an active ingredient. However, the fragrance composition containing the compound represented by Formula (2) as an active ingredient preferably further contains a fragrance in addition to the compound represented by Formula (2).

Note that the term "fragrance composition" refers to a composition to be added to various perfumes and cosmetics, pharmaceutical products, foods, beverages, and the like to impart an aroma to these products, or a composition used per se as a perfume or the like. The fragrance composition may contain an additive, such as a solvent, as necessary in addition to the compound represented by Formula (2) and a fragrance other than the compound represented by Formula (2).

Examples of the additional component contained in the fragrance composition containing the compound represented by Formula (2) as an active ingredient in addition to the compound represented by Formula (2) include fragrances other than the compound represented by Formula (2), surfactants, solvents, antioxidants, and coloring agents. The additional component is preferably at least one selected from the group consisting of fragrances other than the compound represented by Formula (2), surfactants, solvents, antioxidants, and coloring agents, and more preferably at least one selected from the group consisting of fragrances other than the compound represented by Formula (2) and solvents.

The inclusion of a fragrance other than the compound represented by Formula (2) allows the aroma to be adjusted to accommodate a target product. In addition, the inclusion of a solvent allows for easy dissolution in and impregnation of a target product, making it possible to adjust the intensity of the aroma or the durability of the aroma.

The fragrance other than the compound represented by Formula (2) is not limited as long as it is a known fragrance component, and a wide range of fragrances can be used. For example, one, or two or more of the following fragrances can be selected and used at any mixing ratio.

Examples of the fragrance other than the compound represented by Formula (2) include hydrocarbons, alcohols, phenols, esters, aldehydes, ketones, acetals, ketals, ethers, nitriles, lactones, natural essential oils, and natural extracts.

Examples of hydrocarbons include limonene, α-pinene, β-pinene, terpinene, cedrene, longifolene, and valencene.

Examples of alcohols include linalool, citronellol, geraniol, nerol, terpineol, dihydromyrcenol, ethyllinalool, farnesol, nerolidol, cis-3-hexenol, cedrol, menthol, borneol, β-phenylethyl alcohol, benzyl alcohol, phenyl hexanol, 2,2,6-trimethylcyclohexyl-3-hexanol, 1-(2-t-butylcyclohexyloxy)-2-butanol, 4-isopropylcyclohexane methanol, 4-t-butylcyclohexanol, 4-methyl-2-(2-methylpropyl)tetrahydro-2H-pyran-4-ol, 2-methyl-4-(2,2,3-trimethyl-3-cyclopenten-1-yl)-2-buten-1-ol, 2-ethyl-4-(2,2,3-trimethyl-3-cyclopenten-1-yl)-2-buten-1-ol, isocamphylcyclohexanol, and 3,7-dimethyl-7-methoxyoctan-2-ol.

Examples of phenols include eugenol, thymol, and vanillin.

Examples of esters include linalyl formate, citronellyl formate, geranyl formate, n-hexyl acetate, cis-3-hexenyl acetate, linalyl acetate, citronellyl acetate, geranyl acetate, nellyl acetate, terpinyl acetate, nopil acetate, bornyl acetate, isobomyl acetate, o-t-butylcyclohexyl acetate, p-t-butylcyclohexyl acetate, tricyclodecenyl acetate, benzyl acetate, styralyl acetate, cinnamyl acetate, dimethylbenzyl carbinyl acetate, 3-pentyltetrahydropyran-4-yl acetate, citronellyl propionate, tricyclodecenyl propionate, allylcyclohexyl propionate, ethyl-2-cyclohexyl propionate, benzyl propionate, citronellyl butyrate, dimethylbenzylcarbinyl n-butyrate, tricyclodecenyl isobutyrate, methyl-2-nonenoate, methyl benzoate, benzyl benzoate, methyl cinnamate, methyl salicylate, n-hexyl salicylate, cis-3-hexenyl salicylate, geranyl tiglate, cis-3-hexenyl tiglate, methyl jasmonate, methyldihydro jasmonate, methyl-2,4-dihydroxy-3,6-dimethyl benzoate, ethylmethylphenyl glycidate, methyl anthranilate, and fruitate.

Examples of aldehydes include n-octanal, n-decanal, n-dodecanal, 2-methylundecanal, 10-undecenal, citronellal, citral, hydroxycitronellal, dimethyl tetrahydrobenzaldehyde, 4(3)-(4-hydroxy-4-methylpentyl)-3-cyclohexene-1-carboaldehyde, 2-cyclohexyl propanal, p-t-butyl-α-methylhydrocinnamic aldehyde, p-isopropyl-α-methylhydrocinnamic aldehyde, p-ethyl-α,α-dimethylhydrocinnamic aldehyde, α-amylcinnamic aldehyde, α-hexylcinnamic aldehyde, piperonal, and α-methyl-3,4-methylenedioxyhydrocinnamic aldehyde.

Examples of ketones include methylheptenone, 4-methylene-3,5,6,6-tetramethyl-2-heptanone, amylcyclopentanone, 3-methyl-2-(cis-2-penten-1-yl)-2-cyclopenten-1-one, methylcyclopentenolone, rose ketone, γ-methylionone, α-ionone, carbone, menthone, camphor, nootkatone, benzylacetone, anysilacetone, methyl-β-naphthylketone, 2,5-dimethyl-4-hydroxy-3(2H)-furanone, maltol, 7-acetyl-1,2,3,4,5,6,7,8-octalydro-1,1,6,7-tetramethyl naphthalene, muscone, civetone, cyclopentadecanone, and cyclohexedecenone.

Examples of acetals and ketals include acetoaldehyde ethylphenylpropyl acetal, citraldiethyl acetal, phenylacetoaldehyde glycerin acetal, and ethylacetoacetate ethylene glycol ketal.

Examples of ethers include anetol, β-naphthylmethyl ether, β-naphthylethyl ether, limonene oxide, rose oxide, 1,8-cineol, racemic or optically active dodecahydro-3a,6,6,9a-tetramethylnaphtho[2,1-b]furane.

Examples of nitriles include citronellyl nitrile.

Examples of lactones include γ-nonalactone, γ-undecalactone, σ-decaiactone, γ-jasmolactone, cumarin, cyclopentadecanolide, cyclohexadecanolide, ambrettolide, ethylene brassylate, and 11-oxahexadecanolide.

Examples of natural essential oils and natural extracts include those of orange, lemon, bergamot, mandarin, peppermint, spearmint, lavender, chamomile, rosemary, eucalyptus, sage, basil, rose, geranium, jasmine, ylang-ylang, anise, clove, ginger, nutmeg, cardamom, cedar, Japanese cypress, sandalwood, vetiver, patchouli, and labdanum.

Examples of the solvent include dipropylene glycol, diethyl phthalate, ethylene glycol, propylene glycol, methyl myristate, and triethyl citrate.

Examples of the surfactant include polyoxyethylene lauryl sulfate ether.

The fragrance composition containing the compound represented by Formula (2) as an active ingredient can be used as an aroma ingredient for various products.

The products in which the fragrance composition can be used include: fragrance products, such as perfumes and colognes; cosmetics for hair, such as shampoos, conditioners, hair tonics, hair creams, mousses, gels, pomades, and sprays; cosmetics for skin, such as face lotions, serums, creams, emulsions, facial masks, foundations, face powders, lipsticks, and various types of makeup; various detergents for health and sanitation, such as dish detergents, laundry detergents, softeners, disinfectant detergents, odor eliminating detergents, indoor fragrances, furniture care products, glass cleaners, furniture cleaners, floor cleaners, disinfectants, insecticides, bleaches, germicides, and repellents; quasi-drugs, such as toothpaste, mouthwash, bath salts, antiperspirants, and perm solutions; miscellaneous goods such as toilet paper and tissue paper; pharmaceuticals; and food products.

The amount of the fragrance composition added to the products described above is not limited and can be selected depending on the type, nature, and sensory effect of the product to be perfumed. For example, the amount of the fragrance composition added to the products is preferably 0.00001 mass% or greater, more preferably 0.0001 mass% or greater, and even more preferably 0.001 mass% or greater. Also, the amount of the fragrance composition added to the products is preferably 80 mass% or less, more preferably 60 mass% or less, and even more preferably 40 mass% or less.

Note that, when the fragrance composition is used as an aromatic oil, perfume, or the like, the amount of the fragrance composition added to the products may be 80 mass% or greater, or may be 100 mass%.

### <Use of Compound Represented by Formula (2) as Fragrance>

The compound represented by Formula (2) below can be used as a fragrance. 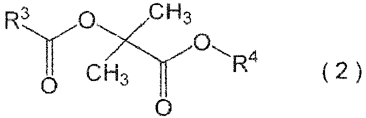 where, in Formula (2), R³ is a linear or branched olefinically unsaturated hydrocarbon group having from 3 to 4 carbons, and R⁴ is a linear, branched, or cyclic alkyl group having from 1 to 6 carbons; however, R³ is not an isopropenyl group.

The compound represented by Formula (2) above has a complex aroma of a floral aroma and another aroma. Thus, the compound represented by Formula (2) above can be used as a fragrance. In particular, the compound represented by Formula (2) above has a floral aroma and, in addition, exhibits aromas having a green tone, a rosy tone, an herbal tone, a woody tone, a Balsamic tone, a fruity tone and the like at the same time depending on R³ or R⁴. Thus, the compound represented by Formula (2) above can be used as a fragrance.

The compound represented by Formula (2) that can be used as a fragrance is the same as the compound represented by Formula (2) described above, and the preferred structures are also the same.

### [Method for Producing Compound Represented by Formula (1) And Method for Producing Compound Represented by Formula (2)]

A method for producing the compound represented by Formula (1) is not limited and is selected as appropriate from commonly known methods. A method for producing the compound represented by Formula (2) is not limited and is selected as appropriate from commonly known methods.

Preferred examples of the method for producing the compound represented by Formula (1) are given below. The method for producing the compound represented by Formula (2) is the method for producing the compound represented by Formula (1) shown below but with "R¹" changed to "R³" and "R²" changed to "R⁴" in Formula (3) and Formula (4).

For example, an isobutyrate ester having an alkenoyloxy group at the α-position [compound represented by Formula (1)] can be produced by reacting an α-hydroxyisobutyrate ester with an unsaturated acid anhydride. The reaction formula for this reaction is Formula (3) below.

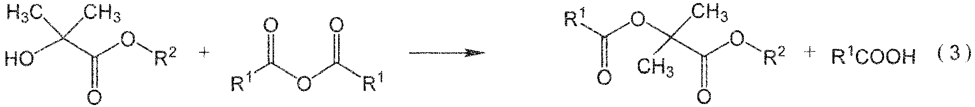

In Formula (3), R¹ is a linear or branched olefinically unsaturated hydrocarbon group having from 2 to 4 carbons, and R² is a linear, branched, or cyclic alkyl group having from 1 to 6 carbons.

Alternatively, the isobutyrate ester having an alkenoyloxy group at the α-position [compound represented by Formula (1)] can be produced by reacting an α-hydroxyisobutyrate ester with an unsaturated carboxylic acid chloride in the presence of a catalyst. The reaction formula of this reaction is Formula (4) below.

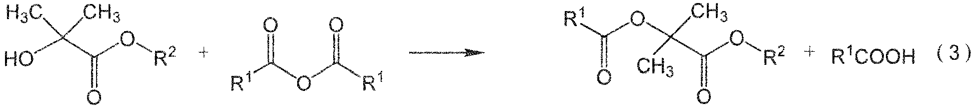

In Formula (4), R¹ is a linear or branched olefinically unsaturated hydrocarbon group having from 2 to 4 carbons, and R² is a linear, branched, or cyclic alkyl group having from 1 to 6 carbons.

For the catalyst, reaction method, reaction conditions, reaction apparatus, and the like, to be used in these reactions, a catalyst, a reaction method, reaction conditions, and a reaction apparatus known in the art can be used without any particular limitation. In addition, a method for purifying the resulting isobutyrate ester having an alkenoyloxy group at the α-position [compound represented by Formula (1) or compound represented by Formula (2)] can be a commonly known purification method.

### Examples

Hereinafter, the present invention will be described in further detail with reference to examples, but the present invention is not limited to these examples.

The reaction performance was evaluated according to the following expression. Reaction yield (%) = [(number of moles of product ester in reaction solution) / (number of moles of raw material ester in solution fed)] × 100%

### [Analysis Method and Separation Method]

### <Gas chromatography analysis (GC analysis)>

Device: "GC-2010" (available from Shimadzu Corporation, product name)
Detector: FID
Column: "DB-1" (capillary column available from J&W Scientific, Inc., product name) (0.25 mmϕ × 30 m × 0.25 µm)

### <NMR spectral analysis>

Identification of the ester was performed by ¹H-NMR measurement and ¹³C-NMR measurement. The measurement conditions are shown below.

Device: "ECA500" (available from JEOL Ltd., product name)
[¹H-NMR]
   Nuclide: ¹H
   Measurement frequency: 500 MHz
   Measurement sample: 5% CDCl₃ solution
[¹³C-NMR]
   Nuclide: ¹³C
   Measurement frequency: 125 MHz
   Measurement sample: 5% CDCl₃ solution

### <Gas Chromatograph-Mass Spectrometry (GC-MS analysis)>

Identification of the compounds was also performed by determining the molecular weight by GC-MS measurement (chemical ionization method [CI+], high resolution mass spectrometry [millimass]). The measurement conditions are shown below.
GC device: "Agilent 7890B" (available from Agilent Technologies, Inc., product name) GC measurement conditions
Column: "DB-1" (capillary column available from J&W Scientific, Inc., product name) (0.25 mmϕ × 30 m × 0.25 µm)
MS device: "JMS-T200 GCx-plus" (available from JEOL Ltd., product name)
MS measurement conditions, chemical ionization method
Ionization voltage: 200V
Ion source: 200°C
Detector voltage: 2100 V
Reagent gas: isobutane

The exact mass values (Exact Mass) of fragments detected in the protonated state by the chemical ionization method and the chemical composition formula thus attributed were described.

### <Product Isolation by Silica Gel Chromatography>

The following materials were used for product isolation by silica gel chromatography in Examples.

Filler: "Wakogel C-200" (available from FUJIFILM Wako Pure Chemical Corporation, product name)

Development solvent: ethyl acetate - hexane

### [Raw Material Synthesis Examples]

### Synthesis Example 1: Synthesis of Isopropyl α-Hydroxyisobutyrate

88.7 g of methyl α-hydroxyisobutyrate (available from Mitsubishi Gas Chemical Company, Inc.), 106.1 g of isopropanol (available from FUJIFILM Wako Pure Chemical Corporation), and 0.21 g of sodium methoxide (available from FUJIFILM Wako Pure Chemical Corporation) were placed in a 300-mL glass flask equipped with a distillation tube. A transesterification reaction was carried out for 48 hours while the mixture was heated under reflux under normal pressure (atmospheric pressure) to remove methanol produced by the reaction from the system. As a result, isopropyl α-hydroxyisobutyrate was produced at a reaction yield of 98.4% by the reaction of Formula (5) below. After water was added to the reaction system to deactivate the catalyst, distillation was performed under reduced pressure, resulting in 77.7 g of isopropyl α-hydroxyisobutyrate (purity by GC analysis, also referred to as GC purity hereinafter: 99.6%) as the fraction at 40 mmHg and 65°C.

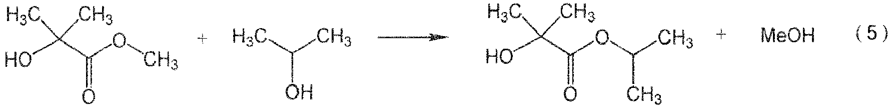

### Synthesis Example 2: Synthesis of Sec-Butyl α-Hydroxyisobutyrate

295 g of methyl α-hydroxyisobutyrate (available from Mitsubishi Gas Chemical Company, Inc.), 203 g of sec-butanol (available from FUJIFILM Wako Pure Chemical Corporation), 2 g of sodium methoxide (available from FUJIFILM Wako Pure Chemical Corporation), and 15 mL of hexane were placed in a 1-L glass flask equipped with a distillation tube. A transesterification reaction was carried out for 18 hours while the mixture was heated under reflux under normal pressure (atmospheric pressure) to remove methanol produced by the reaction from the system through azeotrope with hexane. After water was added to the reaction system to deactivate the catalyst, distillation was performed under reduced pressure, resulting in 160 g of sec-butyl α-hydroxyisobutyrate (GC purity: 99.6%) as the fraction at 78 mmHg and 105°C.

### [Isobutyrate Ester Compound Having Alkenoyloxy Group at α-Position]

### Example 1: Synthesis and Aroma Evaluation of Isopropyl α-Methacryloyloxyisobutyrate

14.6 g of the isopropyl α-hydroxyisobutyrate synthesized in Synthesis Example 1 and 58 mg of iron (III) chloride (available from Alfa Aesar) were placed in a 50-mL glass flask equipped with a stirrer, a condenser, and a pipetting device. While stirring was performed at room temperature, 15.3 g of methacrylic anhydride (available from Tokyo Chemical Industry Co., Ltd.) was slowly added dropwise. After completion of the dropwise addition, the mixture was heated to 60°C and stirred for 12 hours. From a GC analysis of the reaction solution, it was confirmed that isopropyl α-methacryloyloxyisobutyrate was produced at a reaction yield of 81.5% by the reaction of Formula (6) below. Then, a washing operation was performed twice with a 10% aqueous solution of sodium carbonate and once with a saturated aqueous solution of sodium chloride. The washed product was dried over magnesium sulfate and then concentrated, resulting in 12.0 g of isopropyl α-methacryloyloxyisobutyrate (GC purity: 98.3%) produced by silica gel column chromatography. The aroma of the resulting isopropyl α-methacryloyloxyisobutyrate was evaluated by a method described below. Table 1 presents the results of the aroma evaluation.

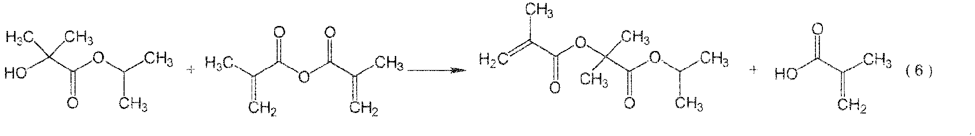

### Examples 2 to 4: Synthesis and Aroma Evaluation of Various α-Alkenoyloxyisobutyrate Esters

With the same reaction device as in Example 1, a reaction was performed using each of α-hydroxyisobutyrate esters (the isopropyl α-hydroxyisobutyrate synthesized in Synthesis Example 1 or the sec-butyl α-hydroxyisobutyrate synthesized in Synthesis Example 2), iron (III) chloride (available from Alfa Aesar) in an amount of 0.03 mol% per α-hydroxyisobutyrate ester, and an acid anhydride corresponding to each of the α-alkenoyloxyisobutyrate esters to be produced in an amount of 1 equivalent relative to each of the α-hydroxyisobutyrate esters. Various α-alkenoyloxyisobutyrate esters were produced by silica gel column chromatography in the same manner as in Example 1. The GC purities, stereoisomer ratios (hereinafter referred to as E/Z ratios), NMR spectrum analysis results, and GC-MS analysis results of the various α-alkenoyloxyisobutyrate esters produced are presented below. For compounds containing stereoisomers, the NMR spectrum analysis results and GC-MS analysis results of the trans (E) stereoisomers are described. The aroma of each of the α-alkenoyloxyisobutyrate esters produced was evaluated by a method described below. Table 1 presents the results of the aroma evaluation.

### Sec-Butyl α-Methacryloyloxyisobutyrate

GC Purity 99.6%

### Isopropyl α-2-Butenoyloxyisobutyrate

GC Purity 99.4% (E/Z = 99/1)
¹H NMR (500 MHz, CDCl₃) δ1.224 (6H, d, J = 6.5 Hz), 1.551 (6H, s), 1.880 (3H, dd, J = 6.5, 1.5 Hz), 5.040 (1H, sept, J = 6.5 Hz), 5.846 (1H, dq, J = 15.5, 1.5 Hz), 6.970 (1H, dq, 3 = 15.5, 6.5 Hz)
¹³C NMR (125 MHz, CDCl₃) δ17.92, 21.50, 24.51, 68.53, 77.93, 122.57, 145.04, 165.13, 172.09
Exact Mass 215.12900 (C₁₁H₁₈O₄, parent peak)

### Sec-Butyl α-2-Butenoyloxyisobutyrate

GC Purity 99.6% (E/Z = 98/2)
¹H NMR (500 MHz, CDCl₃) δ0.837 - 0.932 (3H, m), 1.194 (3H, d, J = 6.5 Hz), 1.537 - 1.601 (2H, m), 1 .556 (3H, s), 1.558 (3H, s), 1.879 (3H, dd, J = 7.0, 1.5 Hz), 4.872 (1H, sext, J = 6.5 Hz), 5.846 (1H, dq, J = 15.5, 1.5), 6.970 (1H, dq, J = 15.5, 7.0)
¹³C NMR (125 MHz, CDCl₃) δ9.53, 17.94, 19.12, 24.57, 24.62, 28.61, 73.14, 78.02, 122.59, 145.09, 165.14, 172.26
Exact Mass 229.14347 (C₁₂H₂₀O₄, parent peak)

### Example 5: Synthesis and Aroma Evaluation of Isopropyl α-(3-Methyl-2-Butenoyloxy)isobutyrate

8.0 g of acetic anhydride (available from FUJIFILM Wako Pure Chemical Corporation) and 23.5 g of 3-methylcrotonic acid (available from Tokyo Chemical Industry Co., Ltd.) were placed in a 100-mL glass flask equipped with a stirrer, a distillation head, and condenser. Under reduced pressure, heating and stirring were performed for 10 hours at 90°C and from 30 to 60 hPa while the resulting acetic acid was being removed from the top of the column, resulting in 3-methylcrotonic anhydride. The liquid temperature was allowed to cool to 60°C. Then, 14.3 mg of iron (III) chloride (available from Alfa Aesar) was dissolved in 11.4 g of isopropyl α-hydroxyisobutyrate synthesized in Synthesis Example 1, and the resulting product was slowly added dropwise to the cooled 3-methylcrotonic anhydride. Heating was carried out at 60°C for 3 hours under normal pressure (atmospheric pressure), resulting in isopropyl α-(3-methyl-2-butenoyloxy)isobutyrate at a reaction yield of 88%. Then, a washing operation was performed twice with a 10% aqueous solution of sodium carbonate and once with a saturated aqueous solution of sodium chloride. The washed product was dried over magnesium sulfate and then concentrated, resulting in 10.0 g of isopropyl α-(3-methyl-2-butenoyloxy)isobutyrate (GC purity: 99.9%) produced by silica gel column chromatography. The NMR spectrum analysis result and GC-MS analysis result of the isopropyl α-(3-methyl-2-butenoyloxy)isobutyrate produced are presented below. The aroma of the isopropyl α-(3-methyl-2-butenoyloxy)isobutyrate produced was evaluated by a method described below. Table 1 presents the results of the aroma evaluation.
¹H NMR (500 MHz, CDCl₃) δ1.225 (6H, d, J = 6.5 Hz), 1.549 (6H, s), 1.893 (3H, d, J = 1.5 Hz), 2.135 (3H, d, J = 1.5 Hz), 5.054 (1H, sept, J = 6.5 Hz), 5.688 (1H, sept, J = 1.5 Hz)
¹³C NMR (125 MHz, CDCl₃) δ20.08, 21.52, 24.59, 27.33, 68.37, 77.38, 115.83, 157.20, 165.27, 172.36
Exact Mass 229.14423 (C₁₂H₂₀O₄, parent peak)

### Examples 6 and 7: Synthesis and Aroma Evaluation of Various Isopropyl α-Alkenoyloxyisobutyrates

With the same reaction device as in Example 5, a reaction was performed using acetic anhydride (available from FUJIFILM Wako Pure Chemical Corporation), the isopropyl α-hydroxyisobutyrate synthesized in Synthesis Example 1, pentenoic acid (available from Tokyo Chemical Industry Co., Ltd.) or tiglic acid (available from Tokyo Chemical Industry Co., Ltd.), which are acid compounds corresponding to the isopropyl α-alkenoyloxyisobutyrate to be produced and used in an amount of 3 equivalents relative to the isopropyl α-hydroxyisobutyrate, and iron (III) chloride (available from Alfa Aesar) in an amount of 0.05 mol% in Example 6 or 0.1 mol% in Example 7 relative to the isopropyl α-hydroxyisobutyrate. Various isopropyl α-alkenoyloxyisobutyrates were produced by silica gel column chromatography in the same manner as in Example 5. The GC purities, E/Z ratios, NMR spectrum analysis results of the trans (E) stereoisomers, and GC-MS analysis results of the trans (E) stereoisomers of the various isopropyl α-alkenoyloxyisobutyrates produced are presented below. The aroma of each of the isopropyl α-alkenoyloxyisobutyrates produced was evaluated by a method described below. Table 1 presents the results of the aroma evaluation.

### Isopropyl α-2-Pentenoyloxyisobutyrate

GC Purity: 99.7% (E/Z = 98/2)
¹H NMR (500 MHz, CDCl₃) δ1.074 (3H, t, J = 7.5 Hz), 1.228 (6H, d, J = 6.5 Hz), 1.55 (6H, s), 2.202 - 2.247 (2H, m), 5.045 (1H, sept, J = 6.5 Hz), 5.819 (1H, dt, J = 15.5, 1.5), 7.022 (1H, dt, J = 15.5, 6.5 Hz)
¹³C NMR (125 MHz, CDCl₃) δ12.00, 21.53, 24.53, 25.26, 68.56, 77.96, 120.13, 151.27, 165.45, 172.13
Exact Mass 229.14404 (C₁₂H₂₀O₄, parent peak)

### Isopropyl α-2-Methyl-2-Butenoyloxyisobutyrate

GC Purity 99.6% (E/Z = 99/1)
¹H NMR (500 MHz, CDCl₃) δ1.220 (6H, d, J = 6.5 Hz), 1.565 (3H, s), 1.568 (3 H, s), 1.792 (3H, dq, J = 7.0, 1.0 Hz), 1.817 - 1.822 (3H, m), 5.041 (1H, sept, J = 6.5 Hz), 6.857 (1H, qq, J = 7.0, 1.5 Hz)
¹³C NMR (125 MHz, CDCl₃) δ11.87, 14.30, 21.51, 24.50, 68.43, 77.91, 128.55, 137.41, 166.78, 172.22
Exact Mass 229.14414 (C₁₂H₂₀O₄, parent peak)

### Example 8: Synthesis and Aroma Evaluation of Isopropyl α-2-Butenoytoxyisobutyrate

3.98 g of 2-butynoic acid (available from Tokyo Chemical Industry Co., Ltd.), 20 mL of diethyl ether (available from FUJIFILM Wako Pure Chemical Corporation), 0.99 g of 5% palladium/barium sulfate (available from Tokyo Chemical Industry Co., Ltd.), and 0.18 mL of quinoline (available from FUJIFILM Wako Pure Chemical Corporation) were placed in a 100-mL glass flask equipped with a stirrer, and stirring was performed at room temperature (25°C) for 1.5 hours under a hydrogen atmosphere. The reaction solution was filtered to remove the catalyst, and the filtrate was concentrated and purified by silica gel column chromatography, resulting in 1.96 g of a mixture containing 84.1% 2-butenoic acid (E/Z=3/97) and 15.9% butanoic acid.

1.82 g of the above mixture containing 2-butenoic acid and 3.75 g of thionyl chloride (available from FUJIFILM Wako Pure Chemical Corporation) were placed in a 100-mL glass flask equipped with a stirrer, and stirring was performed at 50°C for 2 hours. The reaction solution was concentrated to remove unreacted thionyl chloride, and 3.11 g of the isopropyl α-hydroxyisobutyrate synthesized in Synthesis Example 1 was added. Then, stirring was performed at room temperature for 16 hours. Then, the mixture was dissolved in hexane, and a washing operation was performed four times with a 15% aqueous solution of sodium carbonate. The washed product was dried over magnesium sulfate, concentrated, and then purified by silica gel column chromatography, resulting in 1.02 g of a mixture of isopropyl α-2-butenoyloxyisobutyrate [GC purity: 81.4% (E/Z = 29/71)] and isopropyl α-butanoyloxyisobutyrate (GC purity: 17.5%). The NMR spectrum analysis result of the cis (Z) stereoisomer and the GC-MS analysis result of the cis (Z) stereoisomer of the isopropyl α-2-butenoyloxyisobutyrate produced are presented below. The aroma of the resulting isopropyl α-2-butenoyloxyisobutyrate was evaluated by a method described below. Table 1 presents the results of the aroma evaluation.
¹H NMR (500 MHz, CDCl₃) δ1.230 (6H, d, J = 6.0 Hz), 1.568 (6H, s), 2.117 (3H, dd, J = 7.3, 1.8 Hz), 5.057 (1H, sept, J = 6.0 Hz), 5.798 (1H, dq, J = 11.5, 1.8 Hz), 6.344 (1H, dq, J = 11.5, 7.3 Hz)
¹³C NMR (125 MHz, CDCl₃) δ15.31, 21.58, 24.63, 68.55, 77.89, 120.52, 145.50, 165.21, 172.04
Exact Mass 215.12853 (C₁₁H₁₈O₄, parent peak)

### Aroma Evaluation

The aromas of the isobutyrate ester compounds having an alkenoyloxy group at the α-position obtained in Examples 1 to 8 were evaluated by a perfumer, and the results are presented in Table 1.

### [Table 1]

**Table 1**

| | Structural formula | Aroma evaluation |
|---|---|---|
| Example 1 | 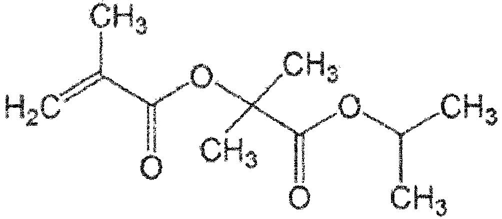 | Floral |
| | | Fruity |
| | | Rosy |
| | | Green |
| | | Herbal |
| Example 2 | 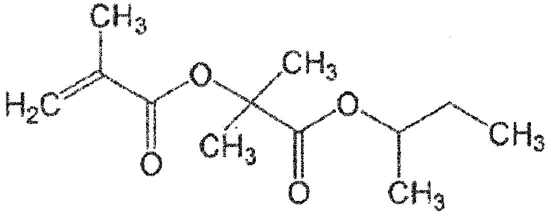 | Green |
| | | Floral |
| | | Herbal |
| | | Metallic |
| Example 3 | 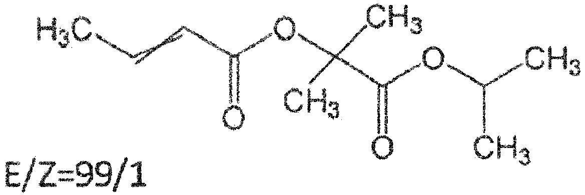 | Floral |
| | | Rosy |
| | | Green |
| | | Woody |
| | | Balsamic |
| Example 4 | 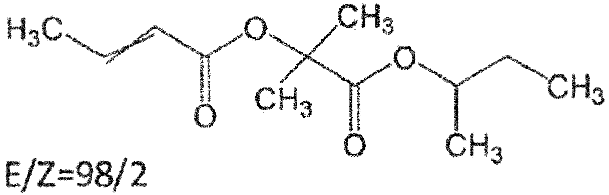 | Floral |
| | | Rosy |
| | | Woody |
| | | Herbal |
| | | Balsamic |
| Example 5 | 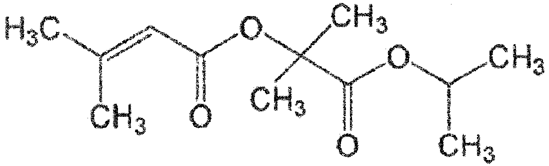 | Floral |
| | | Fruity |
| | | Rosy |
| | | Green |
| | | Herbal |
| Example 6 | 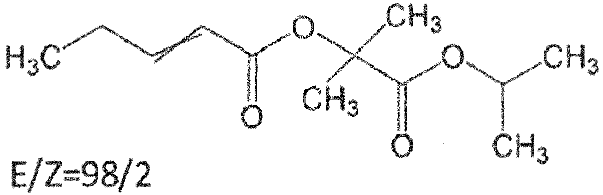 | Woody |
| | | Floral |
| | | Balsamic |
| | | Waxy |
| | | Honey |
| Example 7 | 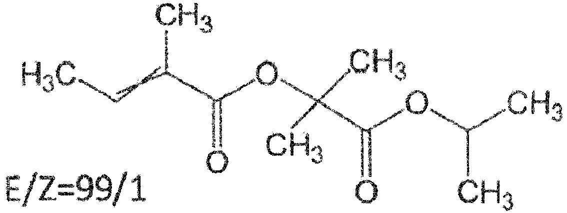 | Floral |
| | | Green |
| | | Balsamic |
| | | Woody |
| | | Fruity |
| Example 8 | 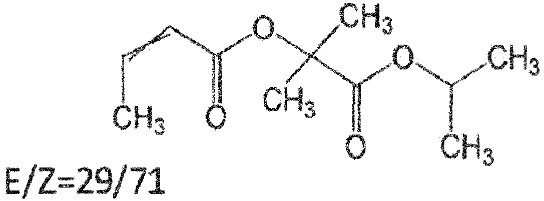 | Floral |
| | | Fruity |
| | | Rosy |
| | | Green |

(The carbon-carbon double bond represented by the crossed double line in the structural formulas of Examples 3, 4, 6, 7, and 8 represents both the trans (E) and cis (Z) stereoisomers resulting from the double bond.)

### Fragrance Composition

### Example 9: Fruity-Herbal-Type Fragrance Composition

A fragrance composition was formulated by adding 35 parts by mass of the isopropyl α-methacryloyloxyisobutyrate obtained in Example 1 to 965 parts by mass of a fragrance composition having the composition presented in Table 2.

According to the aroma evaluation by a perfumer, adding the isopropyl α-methacryloyloxyisobutyrate of Example 1 to the fragrance composition having the composition presented in Table 2 imparted a strong green and fruity sensation. The result was a fruity-herbal-type fragrance composition which can be used for household items such as cleaners.

### [Table 2]

**Table 2**

| Blended components | Parts by mass |
|---|---|
| Aldehyde C12 Lauric | 2.0 |
| Aldehyde C14 | 6.2 |
| Allyl heptanoate | 25.0 |
| Benzyl acetate | 12.0 |
| Benzyl salicylate | 35.0 |
| Camphor | 8.0 |
| cis-3-Hexenol | 0.5 |
| cis-3-Hexenyl acetate | 0.8 |
| Coumarin | 40.0 |
| β Damascone | 2.4 |
| Dihydromyrcenol | 180.0 |
| Dipropylene glycol | 103.5 |
| Ethyl caproate | 1.5 |
| Ethyl-2-methylbutyrate | 1.5 |
| Eucalyptol | 50.0 |
| Isoamyl acetate | 2.0 |
| Isobomyl acetate | 60.0 |
| Linalool | 180.0 |
| Linalyl acetate | 100.0 |
| γ-Methylionone | 2.0 |
| Methyl isoeugenol | 2.6 |
| Orange oil (Florida) | 30.0 |
| Phenylethyl alcohol | 50.0 |
| Terpineol | 25.0 |
| 2-t-butylcyclohexyl acetate | 45.0 |
| Total | 965.0 |

### Example 10: Natural Essential Oil-Like Magnolia-Type Fragrance Composition

A fragrance composition was formulated by adding 100 parts by mass of the isopropyl α-2-methyl-2-butenoyloxyisobutyrate obtained in Example 7 to 900 parts by mass of the fragrance composition having the composition presented in Table 3.

According to the aroma evaluation by a perfumer, by adding the isopropyl α-2-methyl-2-butenoyloxyisobutyrate of Example 7 to the fragrance composition having the composition presented in Table 3, the diffusibility of the aroma was increased, resulting in a blended fragrance with an extended top note. The result was a natural essential oil-like Magnolia-type fragrance composition which can be used as a base fragrance that has a natural feel.

### [Table 3]

**Table 3**

| Blended components | Parts by mass |
|---|---|
| Isoeugenyl acetate | 2.0 |
| Benzyl salicylate | 300.0 |
| Caryophyllene | 30.0 |
| Citronellol | 10.0 |
| Clary sage oil | 5.0 |
| Dipropylene glycol | 219.0 |
| Eucalyptol | 50.0 |
| Geraniol | 10.0 |
| Guaiacum wood oil | 10.0 |
| Isobomyl acetate | 4.0 |
| Lavandin grosso essential | 10.0 |
| Linalool | 100.0 |
| Magnolan | 10.0 |
| Methyl isoeugenol | 4.0 |
| Nerolidol | 20.0 |
| Orange oil (Brazil) | 30.0 |
| α Pinene | 12.0 |
| β Pinene | 14.0 |
| α Terpineol | 50.0 |
| Ylang-ylang complete (Madagascar) | 10.0 |
| Total | 900.0 |

From the results of the Examples, the following is clear: the isobutyrate ester compound having an alkenoyloxy group at the α-position according to an embodiment of the present invention has a complex aroma of a floral aroma and another aroma, and thus is useful as a fragrance; when the isobutyrate ester compound is contained in a fragrance composition as an active ingredient, the resulting fragrance composition is imparted with strength and diffusibility of aroma in addition to the aroma mentioned above.

Therefore, the isobutyrate ester compound having an alkenoyloxy group at the α-position according to an embodiment of the present invention is useful as an active ingredient of a fragrance composition, and the resulting fragrance composition can exhibit desired perfuming properties when added to various products.

In addition, it is clear that the isobutyrate ester compound having an alkenoyloxy group at the α-position according to an embodiment of the present invention has a complex aroma of a floral aroma and another aroma, and thus is useful as a fragrance. It can be seen that the isobutyrate ester compound having an alkenoyloxy group at the α-position according to an embodiment of the present invention has a floral aroma and, in addition, exhibits aromas having a green tone, a rosy tone, an herbal tone, a woody tone, a Balsamic tone, a fruity tone and the like at the same time depending on the unsaturated hydrocarbon group and the alkyl group.

Therefore, the isobutyrate ester compound having an alkenoyloxy group at the α-position according to an embodiment of the present invention is useful as a fragrance and can be suitably used as a fragrance.

## Claims

1. A fragrance composition comprising a compound represented by Formula (1) below as an active ingredient: 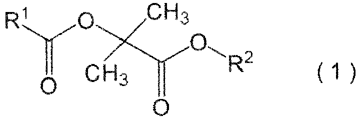 where, in Formula (1), R¹ is a linear or branched olefinically unsaturated hydrocarbon group having from 2 to 4 carbons, and R² is a linear, branched, or cyclic alkyl group having from 1 to 6 carbons.

2. The fragrance composition according to claim 1, wherein, in Formula (1), R¹ is a linear or branched olefinically unsaturated hydrocarbon group having from 3 to 4 carbons.

3. The fragrance composition according to claim 1 or 2, wherein, in Formula (1), R¹ is an isopropenyl group.

4. The fragrance composition according to any one of claims 1 to 3, wherein, in Formula (1), R² is a branched alkyl group having from 3 to 4 carbons.

5. The fragrance composition according to any one of claims 1 to 4, wherein, in Formula (1), R² is an isopropyl group or a sec-butyl group.

6. The fragrance composition according to any one of claims 1 to 5, wherein, in Formula (1), R² is an isopropyl group.

7. The fragrance composition according to claim 1, wherein, in Formula (1), R¹ is an isopropenyl group while R² is an isopropyl group, R¹ is an isopropenyl group while R² is a sec-butyl group, R¹ is a 1-propenyl group while R² is an isopropyl group, R¹ is a 1-propenyl group while R² is a sec-butyl group, R¹ is a 1 -butenyl group while R² is an isopropyl group, R¹ is a 1-methyl-1-propenyl group while R² is an isopropyl group, or R¹ is a 2-methyl-1-propenyl group while R² is an isopropyl group.

8. Use of a compound represented by Formula (1) below as a fragrance: 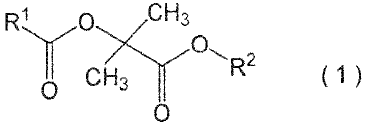 where, in Formula (1), R¹ is a linear or branched olefinically unsaturated hydrocarbon group having from 2 to 4 carbons, and R² is a linear, branched, or cyclic alkyl group having from 1 to 6 carbons.

9. A compound represented by Formula (2) below: 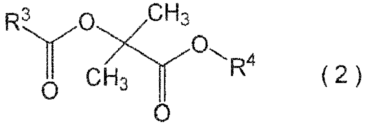 where, in Formula (2), R³ is a linear or branched olefinically unsaturated hydrocarbon group having from 3 to 4 carbons, and R⁴ is a linear, branched, or cyclic alkyl group having from 1 to 6 carbons; however, R³ is not an isopropenyl group.

10. The compound according to claim 9, wherein, in Formula (2), R⁴ is a branched alkyl group having from 3 to 4 carbons.

11. The compound according to claim 9 or 10, wherein, in Formula (2), R⁴ is an isopropyl group or a sec-butyl group.

12. The compound according to any one of claims 9 to 11, wherein, in Formula (2), R⁴ is an isopropyl group.
